# EUROPEAN PATENT APPLICATION

(11) **EP 2 050 437 A1**
(43) Date of publication of application: **22.04.2009**
(21) Application number: 07118508.6
(22) Date of filing: 15.10.2007
(51) Int. Cl.: A61K 9/16, A61K 9/72, A61K 31/195, A61K 31/52, A61K 31/7032

(54) **Improved pharmaceutical dry powder compositions for inhalation.**

(71) Applicant: Laboratoires SMB, 1080 Bruxelles (BE)
(72) Inventor: Vanderbist, Francis, 1650, Beersel (BE); Baudier, Philippe, 1180, Uccle (BE); Amighi, Karim, 1150, Woluwé Saint Pierre (BE); Pilcer, Gabrielle, 2018, Antwerpen (BE)
(74) Representative: Vandeberg, Marie-Paule L.G.

(57) **Abstract**

A spray dried powder for use in dry inhalers, comprising particles (**A**) comprising active material, said particles being at least partially coated **(C₁, C₂)** with an active material so that the flowing and dispersing properties of the powder are enhanced.

## Description

### Field of the invention

The present invention relates to powder compositions for delivery by dry powder inhalers, said powder compositions comprising coated particles of active material. In particular, it relates to powder compositions containing high amounts of active material while still showing enhanced flow properties. It further relates to a method and to a suspension for manufacturing said powder composition.

### Background of the invention

Local delivery of medications to the lung is highly desirable, especially in patients with specifically pulmonary diseases, such as cystic fibrosis, asthma, chronic pulmonary infections or lung cancer, but also, in some cases, to treat non pulmonary diseases, after absorption of the drugs in the respiratory tract and especially in alveoli.

The principal advantages include reduced systemic side effects and higher dose levels of the applicable medication at the site of drug action. Unlike the oral route of drug administration, pulmonary inhalation is not subject to first pass metabolism. Drug inhalation enables a rapid and predictable onset of action, induces fewer side effects than does administration by other routes, and minimizes the risk of drug-drug interactions...

The administration to patients of drugs in the form of fine active particles is already well known and described. Of particular interest to the invention are pulmonary delivery techniques which rely on the inhalation of a pharmaceutical formulation by the patient so that the active drug within the dispersion can reach the distal alveolar regions of the lung. Variability in inspiratory effort may affect the ability of the formulation to be dispersed within a gas stream, the ability to deagglomerate a powdered formulation, and/or the ability of the aerolized formulation to adequately reach the deep lung.

There are a number of different inhalation devices available on the market. The most commonly used device is the pressured metered-dose inhaler (pMDI), but one major limitation of drug delivery to the lungs by a pMDI is the relatively poor efficacy of these devices. Pressurized metered-dose inhalers usually provide much smaller unit doses than would be considered practical for antibiotic therapy or drugs active at high concentration levels. Moreover, several studies have shown the coordination difficulties met by the patients.

Another known inhaler is based on Nebulization which has many well-documented disadvantages, including extended administration time, high cost, low efficiency and poor reproducibility, risk of bacterial contamination, and the need for bulky compressors or gas cylinders. These preparations normally contain antioxidant and preservatives which are known to cause paradoxical reactions such as bronchospasm and cough.

A different approach of delivering drugs to the lung is the formulation of a dry powder for inhalation which is activated and driven by patients' inspiratory flow. In some case they deliver even a higher amount of drug to the lungs leading to the conclusion that lower drug doses delivered with certain dry powder inhalers (DPI) devices are as effective as pMDIs. Administration of antibiotic or other active drug using dry powder aerosols to the lung has been attempted, but studies were limited by inefficient delivery devices and/or poorly dispersible lactose formulations. Preparing dry powder formulations for inhalation is an attractive and appreciated proposition because many solubility and stability issues can be avoided. Further advantages are the low susceptibility to microbial growth and the suitability for both water soluble and insoluble drugs.

However, the small particle size necessary to achieve efficient lung deposition causes problems in processing (poor flowability) and redispersion (strong agglomeration and adhesion). The percentage of the emitted dose deposited in the lungs is dependent on the powder dispersibility, which is limited by interparticular attractive forces. Strong interparticulate forces result in poor powder flow, as well as in poor powder dispersion from passive DPI devices, which results in decreased drug deposition in the lung. These forces are proportional to the area of contact and the separation distance between the particles.

The micronization process leads to small, irregularly shaped flat particles. The smaller the particles are, the stronger are the cohesive forces. Additionally, extensive flat surfaces promote large contact areas, resulting in increased adhesion between the particles. Micronized powders with high energetic surfaces show poor flow properties.

The deposition site and the efficiency of inhaled aerosols in the respiratory tract are critically influenced by the dispersion properties of the particles, and the aerodynamic diameter, size distribution, shape and density of generated particles.

In order to reach the lower respiratory tract and optimize systemic drug absorption, dry powder aerosols need to present aerodynamic diameters of between 1 and 5 µm. Particles that are larger than this range impact in the oro-pharynx while sub-micron particles remain suspended in air and are exhaled. Since micronized drug particles are generally very cohesive and characterized by poor flowing properties, they are usually blended, in dry powder formulations, with coarse and optionally fine carrier particles. These carrier particles are generally carbohydrates, mainly mannitol and lactose, which are approved by the Food and Drug Administration (FDA).

For an effective lung deposition, it is necessary to add great amounts of excipients to the active particles. The weight ratio of excipients to said active compound(s) can be about 50/50 or about 70/30 or about 80/20 or about 90/10 or about 99/1 and even about 99.9/0.1. Furthermore, the carrier particles should be chemically and physically stable, inert to the drug substance and should not exhibit harmful effects, especially on the respiratory tract. In fact, the number of carriers or fillers acceptable for inhalation purpose is very limited because of many different requirements to meet before they are used.

For drugs active at high doses, it is important to have excipients which are effective at low doses or to modify the physical properties (shape, surface morphology, contact area, roughness, hygroscopicity...) of the active compound to increase the aerolisation, flowabiliy and desagglomeration properties and to allow a high dose of active ingredient per inhaled puff.

The increased efficiency of redispersion of the fine active particles from the agglomerates or from the surfaces of carrier particles during inhalation is regarded as critical step in improving the efficiency of the dry powder inhalers. It is known that the surface properties of active compound are important. The shape and texture of the active compound should be such as to give sufficient adhesion force to hold the active particles to the surface of the carrier particles during preparation of the dry powder and in the delivery device before use, but that force of adhesion should be low enough to allow the dispersion of the active particles in the respiratory tract.

U.S. Pat. No. 6,582,728 describes a method for preparing a pharmaceutical dry powdered composition by utilizing spray-dried system by converting an aqueous solution of mixture of active material and excipient into dry particles. The particles of the powders thus obtained consist of a blend of active material and excipients.

U.S. Pat. Appl. No. 2005/0152849 describes a core-coat type dry powder comprising active particles coated with excipients. The fraction of active compound contained in said powders is usually limited by the relatively important amount of excipient required to enhance flow properties.

It is highly desirable -albeit difficult- to obtain powder compositions combining high contents of active material and enhanced flow properties. The present invention allows these usually incompatible properties to be optimized in combination.

### Summary of the invention

The present invention is defined in the appended independent claims. In particular, the present invention provides a new formulation of drug powder containing no excipient or a very limited amount of excipients, for instance less than 10% (weight/weight) compared to the total weight of the powder composition and still yielding enhanced flow properties, thus allowing the delivery of a high dose of active drug to the lungs.

These new powder formulations based on the use of very low excipient levels and presenting very high lung deposition properties offer very important perspectives in improving the delivery of drugs to the pulmonary tract.

These formulations are more particularly useful for drugs that are active at relatively high doses as they permit the delivery of a high concentration of drug (i.e. antibiotic, mucolytic, ...) directly to the site of action while minimizing systemic exposition. A reduction in administration time and in systemic side effects allows improved use and comfort of these formulations for patients.

It is an object of the present invention to provide a powder composition comprising particles comprising active material, said particles being at least partially coated with an active material so that the flowing and dispersing properties of the powder are enhanced.

The powder compositions of the present invention are particularly well-tolerated and well suited for inhalation and efficacious delivery of drugs into the endobronchial space for treating respiratory and non respiratory diseases. A composition of the invention can be used with micronized active compounds in order to promote the release of the active particles from the carrier particles on the actuation of the inhaler, improving the drug deposition. More particularly, a composition of the invention consists of solid particles of the core-coat type, each particle being uniform in structure. Advantageously, in a composition according to the invention, the particles have a mean diameter of 0.5 µm to 10 µm.

A powder composition according to the invention can be used for treating respiratory diseases, wherein said active compound is a drug (i.e. a medicament or pharmaceutically active compound) for such diseases.

A composition according to the invention further comprises at least one active compound in particulate form. Said active compound(s) can be selected (but is not limited to) from the group consisting of:
-Antibiotics such as β-lactam agents (flucloxacillin, cloxacillin, ampicillin, amoxicillin, cefadroxil, cefaclor, cefuroxim, ceftazidim, ceftriaxone, meropenem, imipenem...), macrolids (erythromycin, azithromycin, clarithromycin, roxithromycin, miocamycin, spiramycin), tetracyclin (doxycyclin, minocyclin), aminoglycosids (amikacin, gentamycin, spectinomycin, tobramycin), glycopeptides (vancomycin), ryfamycin's (rifabutin, rifamycin), clyndamycin, quinolon's (ciprofloxacin, levofloxacin, moxifloxacin, norfloxacin, ofloxacin) or a mixture of thereof.
-Anti-histaminic, anti-allergic agents, and any antimicrobial agents, antiviral agents, anticancer agents, antidepressants, antiepileptics, antipains, anti-COPD-agents, anti-asthmatics
-cromones, in particular sodium cromoglycate and nedocromil,
-mucolytic, in particular nacystelyn, n-acetylcystein, mesna, carbocystein,
-leukotriènes, leukotriene antagonist receptors,
-muscle relaxants, hypotensives, sedatives,
- therapeutic proteins, peptides and genes, (poly)peptides, in particular DNase, insulin, cyclosporine, interleukins, cytokines, anti-cytokines and cytokine receptors, vaccines, leuprolide and related analogues, interferons (interferon-alpha, interferon-beta, interferon-gamma or interferon-omega), growth hormones, colony stimulating factors, TNFs, blood factors, enzymes, antibodies to treat immune diseases, virus infections, alpha-1-antitrypsin, proteoglycans such as heparin, genes, desmopressin, immunoglobulins, erythropoietin, calcitonin and parathyroid hormone. The composition of the present invention can also comprise two or more acitve ingredients. More particularly, said active compound comprises or consists of tobramycin, clarithromycin, cromoglycate, nacystelyn or combination thereof.

Formulations can be composed of tobramycin in combination with another active drug such as another antibiotic or amiloride.

Advantageously, the composition of the invention comprises excipients, with weight ratios of excipients to said active compound(s) comprised between (about) 20:80 and (about) 0:100, preferably (about) 5:95 or more preferably (about) 2:98 and even more preferably (about) 0.1:99.9 or 0:100 (no excipient).

A composition according to the invention, comprising said active compound(s), is (for use as) a medicament for treating humans. A composition of the invention may also contain any acceptable pharmacologically inert material or combination of materials. For example, sugar alcohols; polyols such as sorbitol, mannitol and xylitol, and crystalline sugars, including monosaccharides (glucose, arabinose) and disaccharides (lactose, maltose, saccharose, dextrose); inorganic salts such as sodium chloride and calcium carbonate; organic salts such as sodium lactate; other organic salts such as urea, polyssacharides (starch and its derivatives); oligosaccarides such as cyclodextrins and dextrins; sorbitan esters (for example SPAN 85); aerosil 200; magnesium stearate; lecithin; amino acids (leucine, isoleucine, lysine, valine, methionine, phenylalanine); derivative of an amino acid (acesulfame K, aspartame); lauric acid, palmitic acid, stearic acid, erucic acid, behenic acid, or derivatives (such as esters and salts); sodium stearyl fumarate, sodium stearyl lactylate; phosphatidylcholines, phosphatidylglycerols and other examples of natural and synthetic lung surfactants, lauric acid and its salts, for example, sodium lauryl sulphate, magnesium lauryl sulphate, triglycerides, sugar esters; phospholipids, cholesterol; talc; titanium dioxide, aluminium dioxide, silicone dioxide and starch.

Preferably, the excipients are mono-, di- or polysaccharides, among which, use is preferably made of one of the two diglucosides lactose and trehalose, especially lactose.

Advantageously, the excipients comprise physiologically acceptable material. Clearly, it is highly preferable for the excipient to be a material which may be safely inhaled into the lower lung, where it would usually be absorbed into the blood stream. The excipient should therefore be one which is safe to administer by inhalation. The additive material may include a combination of one or more materials.

Advantageously, the composition can be composed of 100% of the active drug, what means that it does not contain any carrier excipients. The additive material can be considered as particles of the active drug with another particle size. The present invention can also consist in a mixture of different particle size of the active drug. For example, a composition of the invention can consist in a mixture of microparticles and nanoparticles of active drug or of a combination of active drugs. Coating of the fine drug particles with particles in the nanometer range can reduce Van Der Waals forces and powder agglomeration. One microparticle can be completely covered with a single layer -or alternatively with several layers- of nanoparticles.

Advantageously, the fraction of microparticles consists of particles having a mean particle size between about 0.5 and 10 µm, preferably in the range of about 1 µm to about 5 µm. Advantageously, the fraction of nanoparticles consists of particles having a mean size between about 0.001 and 1 µm, preferably in the range of about 0.010 µm to about 0.060 µm.

Advantageously, in a composition of the invention, said active compound(s) can be in a particularly high content. More particularly, said weight ratio of nanoparticles to said microparticles can be comprised between (about) 30:70 and (about) 0:100, preferably is (about) 20:80 or more preferably is (about) 10:90 and even more preferably (about) 5:95.

In order to modify the physical properties of the powder (surface morphology, shape, size, density, roughness, contact area...), the active compound can be homogenized with a high speed homogenizer, with high pressure homogenization, ultra-sound or jet mill...

A method for preparing a composition according to the invention comprises the steps of (a) preparing a composition comprising (i) particles in suspension in a liquid carrier, said particles containing at least one active compound and (ii) a coating material comprising active material in suspension or solution in said liquid carrier, and (b) spray drying said composition from step (a) to produce a dry powder composition wherein said particles of active compound are at least partially coated with said coating material.

Spray-drying has been recognized as a successful process to generate powders from solutions in a single step. It converts a liquid feed (solution, coarse suspension, colloidal dispersion) to a dried particulate form. The principal advantages with respect to pulmonary drug delivery are the ability to manipulate and control mean particle size and particle size distribution, particle shape and density in addition to macroscopic powder properties such as bulk density, flow ability, and dispersibility.

A typical Spray-Dryer allows the recovery of a range of particle size going from 0.5 to 30 µm. The lower limitation is given by the separation of the cyclone used: smaller particles can not be separated anymore and are going into the filter.

A method of the invention can further comprise the steps of:
- reducing the size of the particles with high pressure homogenization,
- using jet-mill apparatus to modify the size and surface properties of the particles,
- heating said solution or suspension,
- in case of a suspension, homogenizing said suspension,
- spray drying the said solution or suspension.

Advantageously, a composition of the invention consists of particles having a mean size between about 0.2 µm and 50 µm, preferably in the range of about 0.2 µm to about 20 µm, and more preferably in the range of about 0.5 µm to about 10µm, or even more preferably in the range of about 1 µm to about 5 µm.

A composition according to the invention can be used for the manufacture of a medicament with the advantages of:
- improving the delivery and the uniformity of delivery (between doses) of a high dose of said active compound;
- improving the lung drug deposition of said active compound;
- promoting the dispersal of said active compound, forming an aerosol on actuation of said inhaler;
- improving said active compound fine particle dose value;
- improving the systemic and local tolerance to said active compound after inhalation;
- treating a respiratory disease or a non respiratory disease.

This invention proposes the possibility to obtain different compositions for pulmonary administration having satisfactory properties in term of increasing drug deposition.

### Definitions

The following terms used to describe the present invention have the following meanings. The term "active" in the expressions: "active material", "active ingredient", "active compound" or "active particle", means "pharmaceutically active".

The term "nanoparticles" means solid discrete particles ranging in size from 1 nm to 1000 nm.

The terms "excipient", "carrier material", "carrier particles", "glidant material" or "additive material" all cover pharmaceutically acceptable, non-active compounds.

### Brief description of the drawings

Figure 1 shows the ideal models of active particles with (a) continuous film coating, (b) nanoparticles coating and (c) continuous film containing nanopaticles. In this figure, (**A**) represents an active particle and (**C**) represents a coating, (**C₁**) being a coating in the form of a continuous film and (**C₂**) in the form of nanopaticles.
Figure 2 shows the X-ray powder diffraction patterns of the micronized tobramycin and the spray-dried tobramycin.
Figure 3 shows laser diffraction particle size distribution and undersize curve of spray-dried formulation measured with the Mastersizer 2000^{®}.
Figure 4 shows average particle size distribution and undersize curve measured with the Spraytec^{®} of a) Micronized tobramycin b) Spray-dried tobramycin.
Figure 5 shows SEM (scanning electron microscopy) microphotographs, at different magnifications of micronized tobramycin and spray-dried tobramycin. a) Micronized tobramycin magnification 50x b) Micronized tobramycin magnification 5000x c) spray-dried tobramycin magnification 200x d) spray-dried tobramycin magnification 5000x.
Figure 6 shows laser diffraction particle size distribution of the nanoparticles fraction of spray-dried nanoformulation measured with the Mastersizer^{®} (Hydro 2000).
Figure 7 shows the in vitro comparative deposition of Tobra Form 2: before labelling, after labelling (drug) and after labelling (radioactive) (n=3).
Figure 8 represents scintigraphic images (of the same patient) obtained using the Aerolizer loaded with 25 mg of tobramycin DPI formulation (left inside) and the Tobi® (nebulizer solution, 300 mg)(right inside).
Figure 9 represents mean plasma concentrations of obramycin normalized for lung dose following administration of tobramycin DPI formulation and nebulized tobramycin (Tobi^{®}) (mean ± S.E.M, n = 9)

### Detailed description of the invention

The delivery of dry powder particles of pharmaceutical products to the respiratory tract presents certain problems. The inhaler should deliver the maximum possible proportion of the active particles to the lungs, including a significant proportion to the lower lung, preferably even at the low inhalation capabilities to which some patients with chronic respiratory diseases, especially cystic fibrosis patients, are limited.

It has been found, however, that when currently available dry powder inhaler devices are used, in many cases only about 10 % to 30% of the active particles that leave the device on inhalation are deposited in the lower lung. More efficient dry powder inhalers would clearly bring clinical benefits.

At exit from the inhaler device, the active particles should be physically and chemically stable and should remain in suspension until they reach a conducting bronchiole or smaller branching of the pulmonary tree or other absorption site preferably in the lower lung. Once at the absorption site, the active particle should be capable of efficient collection by the pulmonary mucosa with no active particles being exhaled from the absorption site.

The size of the active particles is particularly important. For effective delivery of active particles deep into the lungs, the active particles should be small, with an equivalent aerodynamic diameter substantially in the range of 1 to 5 µm, approximately spherical and monodispersed in the respiratory tract.

Small particles are, however, thermodynamically unstable due to their high surface area/volume ratio, which provides significant excess surface free energy and encourages particles to agglomerate.

In the inhaler, agglomeration of small particles and adherence of particles to the walls of the inhaler are problems that result in the active particles leaving the inhaler as large stable agglomerates or being unable to leave the inhaler and remaining adhered to the interior of the inhaler.

In an attempt to improve that situation, formulations for use in dry powder inhalers often include coarse carrier particles optionally mixed with fine particle of active material.

The active particles adhere to the surfaces of the carrier particles whilst in the inhaler device, and are dispersed on inhalation into the respiratory tract to give a fine suspension. The carrier particles are often large particles greater than 50 µm or preferably greater than 90 µm, in diameter to give good flow properties because smaller particles may become coated on the wall of the delivery device and have poor flow properties leading to poor dose uniformity.

There are, however, problems associated with the addition of carrier particles to the active particles in the dry powder, for example problems related to the efficient release of the active particles from the surface of the carrier particles on inhalation. Moreover, to obtain good deposition results, it is necessary to add great amount of excipients to the active particles. The weight ratio of excipients to said active compound (s) can be about 80/20 or 90/10 or even about 99/1.

Furthermore, in some cases it is preferred to avoid the use of particles of carrier or to limit its amount as much as possible in the powder administered, especially for drug substances such as antibiotics that are active at high dose ranges.

Problems involved in dispensing a powder containing only particles of active material include:
- formation of stable agglomerates of the small particles which often are not broken down into individual particles in the air stream when the particles are inhaled and are, therefore, less likely to reach the lower lung on inhalation of the powder than the fine individual active particles;
- variations in the amount of powder metered from a reservoir of the inhalation device due to poor flow properties of the powder and inconsistent agglomeration, leading to inconsistency in the size of dose, which may vary as much as 50% compared with the nominal dose for the device;
- incomplete removal of the dose from the device due to adherence of the particles to the walls of the device, leading to poor dose reproducibility.

A purpose of the invention is to prevent and avoid the formation of stable agglomerates of the active material in the powder.

As indicated above, stable agglomeration of the active particles with the known powders may lead to decrease deposition of the active material in the deep lung, together with poor dose uniformity. That is because, when the small active particles agglomerate, the agglomerates which are formed may have a large diameter of more than 20 µm even 100 µm or more. If those agglomerates do not break up when the powder is inhaled, they are unlikely to reach the deep lung due to their size.

Therefore the formation of easily dispersible agglomerates of particles in the powder may be a good solution.

In a composition of the invention, redispersible or reversible agglomerates can be produced by the addition of very low excipients levels in order to significantly modify particles surface properties and reduce their tendency to agglomeration.

A suspension of the active particles must be made in an appropriate solvent where the active particles are poorly soluble while the coating material may dissolve at least partially in it and coat the micron- or nanometer-sized particles upon spray-drying (for example: tobramycin in isopropanol or budesonide in water).

The coating material can also be poorly soluble in the solvent used and be present in the form of nanoparticles of at least one active compound or excipient which can cause efficient reduction of the inherent agglomeration tendency of the active microparticles.

Advantageously, the fraction of microparticles consists of particles having a mean size between about 0.1 and 10 µm, preferably in the range of about 1 µm to about 5 µm. The measure of the particle size is preferably performed using the laser diffraction technology.

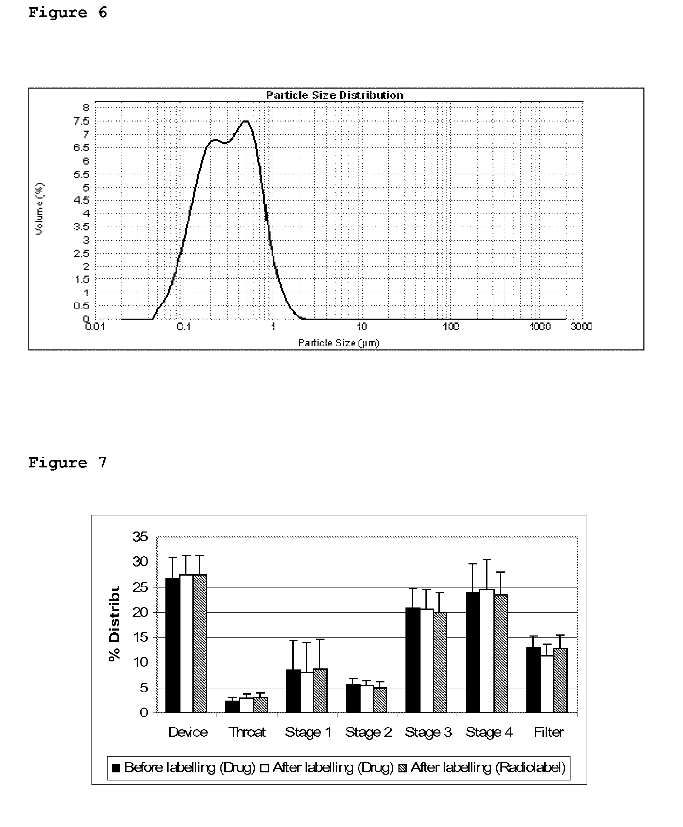

Advantageously, the fraction of nanoparticles consists of particles having a mean size between about 0.001 and 1 µm, preferably in the range of about 0.002 µm to about 0.5 µm. The particle size distribution of the nanoparticles may be performed using the zeta potential technique. For example, Figure 6 shows nanoparticle having a narrow particle size distribution comprised in the range from 50 nm to 1.1 µm, with a d(0.5) about 300 nm.

Advantageously, in a composition of the invention, said active compound(s) can be in a particularly high content such as more than 80 % (w/w) in comparison to the powder composition, preferably superior to 90 %(w/w) in comparison to the powder composition, or even of 100% (without any excipient). More particularly, said weight ratio of nanoparticles to said microparticles can be comprised between (about) 30:70 and (about) 0:100, preferably is (about) 20:80 or more preferably is (about) 10:90 and even more preferably (about) 5:95.

In a composition of the invention, it is also possible to modify the surface properties of the particles by coating the particle of active compound with a continuous film of at least one active material or excipient dissolved in the solvent system. The solvent system can contain more than one solvent. It can be a mixture of different solvents and co-solvents.

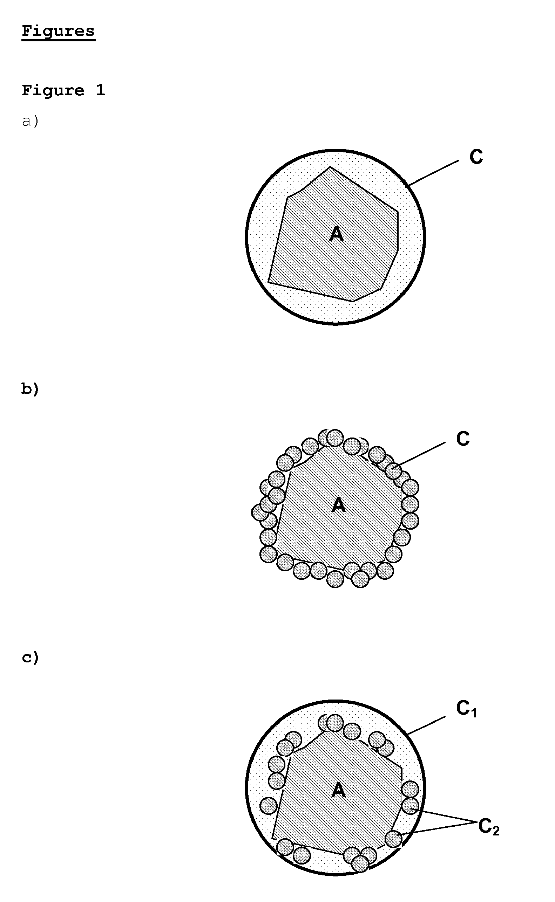

It is important to significantly modify the surface properties and the size of the particles. The coating of the active compound is an alternative permitting the modification of the surface properties of the particles, increasing the flowability, the desagglomeration tendency and the fine particle fraction in the deep lung. The particles of active compound of the present invention may be (at least partially) coated with a continuous film (cf. fig. 1(a)), with nanoparticles adhering to their surfaces (cf. fig. 1(b)), or with nanoparticles embedded in a continuous coating film (cf. fig. 1(c)). The coating material may contain or consist of excipients.

In a composition of the invention, the active compound(s) can be any drug, preferably a drug which is usually administered nasally or orally, in particular by inhalation, e.g. for the treatment of respiratory diseases.

The active compound(s) can also be any drug(s) that can be administered nasally or orally by inhalation in order to reach the systemic circulation and treat respiratory or non respiratory diseases.

A composition according to the invention can be used for treating respiratory diseases, wherein said active compound is a drug (i.e. a medicament or pharmaceutically active compound) for such diseases.

A composition according to the invention can further comprise at least one active compound in particulate form. Said active compound(s) can be selected from the group consisting of:
- Antibiotics such as β-lactam agents (flucloxacillin, cloxacillin, ampicillin, amoxicillin, cefadroxil, cefaclor, cefuroxim, ceftazidim, ceftriaxone, meropenem, imipenem...), macrolids (erythromycin, azithromycin, clarithromycin, roxithromycin, miocamycin, spiramycin), tetracyclin (doxycyclin, minocyclin), aminoglycosids (amikacin, gentamycin, spectinomycin, tobramycin), glycopeptids (vancomycin), ryfamycin's (rifabutin, rifamycin), clyndamycin, quinolon's (ciprofloxacin, levofloxacin, moxifloxacin, norfloxacin, ofloxacin) or a mixture of thereof.
- Anti-histaminic, anti-allergic agents, antibiotics and any antimicrobial agents, antiviral agents, anticancer agents, antidepressants, antiepileptics, antipains,
- cromones, in particular sodium cromoglycate and nedocromil,
- mucolytic, in particular nacystelyn, n-acétylcystéine, mesna, carbocystein
- leukotrienes, leukotriene antagonist receptors,
- muscle relaxants, hypotensives, sedatives,
- therapeutic proteins, peptides and genes, (poly)peptides, in particular DNase, insulin, cyclosporine, interleukins, cytokines, anti-cytokines and cytokine receptors, vaccines, leuprolide and related analogues, interferons (interferon-alpha, interferon-beta, interferon-gamma or interferon-omega), growth hormones, colony stimulating factors, TNFs, blood factors, enzymes, antibodies to treat immune diseases, virus infections, alpha-1-antitrypsin, proteoglycans such as heparin, genes, desmopressin, immunoglobulins, erythropoietin, calcitonin and parathyroid hormone.

More particularly, said active compound comprises or consists of tobramycin, cromoglycate, nacystelyn or a macrolide derivative. Formulations can comprise a combination of two or more active ingredients.

Advantageously, in a composition of the invention, said active compound(s) can be in a particularly high content. More particularly, said weight ratio of excipients to said active compound(s) can be comprised between (about) 10:90 and (about) 0:100, preferably is (about) 5:95 or more preferably is (about) 2:98 and even more preferably (about) 0.1:99.9 or 0:100 (no excipient).

Said formulation, containing high doses of antibiotic, can be advantageously used for the treatment and prophylaxis of bronchiectasis or acute and chronic endobronchial infections, in particular those caused by the bacterium Pseudomonas aeruginosa associated to lung diseases such as cystic fibrosis (CF).

Chronic airway infections are closely associated with progressive deterioration in lung function of cystic fibrosis patients and subsequent mortality in adolescents and adults: patients lose an average of 2 % of their lung function per year and about 90 % of all CF patients die due to progressive pulmonary disease.

The aim of antibiotic therapy in the chronically-infected CF patients is to stabilise lung function and, if possible, to restore some of the lost lung function.

Bronchiectasis can be caused by either acquired or congenital mechanisms that disrupt the normal processes of airway clearance and/or host defense. These causes may include ciliary motility disorders and cystic fibrosis, or processes that cause persistent damage, such as bacterial or viral pneumonia.

Clinically, bronchiectasis is characterized by chronic airway infection, associated with intermittent exacerbations. Infection may manifest by expectoration of purulent sputum, fever, malaise, and weight loss. The bactericidal activity of tobramycin is accomplished by binding irreversibly to 30S and 50S ribosomal subunits resulting in a defective protein.

As aminoglycosides are highly polar, a poor drug penetration into the endobronchial space is generally observed when the parenteral route of administration is used. The mean peak sputum concentration after parenteral administration is only 12 to 20 % of the peak serum concentration.

Like other aminoglycosides, tobramycin has a comparably narrow safety margin. The therapeutic plasma concentration of tobramycin is in the range of 4-8 mg/l and may cause severe ototoxicity and nephrotoxicity in a long-term therapy.

The administration of aminoglycosides by inhalation offers an attractive alternative, delivering high concentrations of antibiotic directly to the site of infection while minimizing systemic bioavailability

Advantageously, the particles of the composition of the invention have a mean particle size smaller than about 50 µm, preferably smaller than about 20 µm, and more preferably smaller than about 10 µm, 5 µm, or even smaller than about 1 µm, or 0.2 µm.

The size of the particles may be evaluated by using laser diffraction or any other standard methods of particle sizing or by sizing methods allowing the determination of the aerodynamic diameter of particles according to the methods described in the European or US Pharmacopeas.

When the use of micronized drug (or active ingredient(s)) is required, the micronized drug particles might have a mean particle size lower than about 20 µm, preferably lower than about 5 µm, such as about 2 µm or about 3 µm. For example, at least 95 % by weight of active particles can have a size lower than 5 µm.

In order to avoid strong agglomeration and to deliver high doses of the active drug, it is possible to allow the:
- addition of nanoparticles produced by high pressure homogenization (Homogenization is a fluid mechanical process that involves the subdivision of particles or droplets into micron sizes to create a stable dispersion or emulsion for further processing. It prepares feeds so that subsequent spray drying produces the best quality of powders.)of the active compound or of the excipient,
- addition of small amounts of a co-solvent in order to dissolve a fraction of the active compound or of the excipient for coating the crystalline particles
- addition of excipients in very small amount to decrease strong agglomeration

Excipients may be present in the powder both in form of small (nano-) particles and in form of a coating on the surfaces of the particles of active compound. Furthermore, because the excipients will in many cases be inhaled into the lung, it is preferable to use only a small amount of additive material. The optimum amount of excipients in the powder will depend on the active compound and excipient used.

Advantageously, the powder comprises not more than 10 % by weight, preferably not more than 5 % by weight, of excipients. In some cases it will be advantageous for the powder to contain about 1 % by weight of excipients or even no excipient at all.

The invention also provides a particularly high dose of active ingredient in the powder composition. The dose in active ingredient being of more than 5 mg of the powder per one actuation of the inhaler, preferably more than 10 mg, and more preferably more than 20 mg. Obviously, the level of the dose will depend on the active material to be delivered, the inhaler device used and the therapeutic target.

The carrier material can be selected from a wide variety of materials which are preferably known to be suitable for DPI, including monosaccharides (eg. glucose fructose, mannose and arabinose), disaccharides (eg. lactose, saccharose and maltose), polyols derived from these disaccharides, such as lactitol, mannitol, or their monohydrates ; polysaccharides (eg. sorbitol, mannitol and xylitol or their monohydrates), inorganic salts such as sodium chloride and calcium carbonate; organic salts such as sodium lactate; other organic salts such as urea, polyssacharides (starch and its derivatives); oligosaccarides such as cyclodextrins and dextrins; sorbitan esters (for example SPAN 85); aerosil 200; magnesium stearate; lecithin; amino acids (leucine, isoleucine, lysine, valine, methionine, phenylalanine); derivative of an amino acid (acesulfame K, aspartame); lauric acid, palmitic acid, stearic acid, erucic acid, behenic acid, or derivatives (such as esters and salts); sodium stearyl fumarate, sodium stearyl lactylate; phosphatidylcholines, phosphatidylglycerols and other examples of natural and synthetic lung surfactants, lauric acid and its salts, for example, sodium lauryl sulphate, magnesium lauryl sulphate, triglycerides, sugar esters; phospholipids, cholesterol; talc; titanium dioxide, aluminium dioxide, silicone dioxide and starch or a mixture of thereof.

Preferably the carrier material is a crystalline sugar such as glucose, lactose, fructose, mannitol or sucrose because such sugars are both inactive and safe. Most preferably, lactose is used.

The addition of excipients decreases the cohesion between the particles of the powder containing the active compound. It is thought that the excipients interfere with the weak bonding forces, such as Van der Waal's and Coulomb forces, between the small particles, helping to keep the particles separated and may be thought of as weak links or "chain breakers" between the particles. Adhesion of the particles to the walls of the device is also reduced in presence of carrier. Where agglomerates of particles are formed, the addition of the additive material decreases the stability of those agglomerates so that they are more likely to break up in the turbulent airstream created on inhalation to form small individual particles which are likely to reach the lower lung.

It is also advantageous for as little as possible of the excipients to reach the lungs on inhalation of the powder. Although the excipients will most advantageously be one that is safe to inhale into the lungs, it is still preferred that only a very small proportion, if any, of the excipients reaches the lung, in particular the lower lung.
Advantageously another excipient could be added and act as an anti-adherent material which will tend to decrease the cohesion between the active compound and the carrier particles such as Aerosil 200, leucine, lecithin, Sorbitan ester (SPAN 85), Magnesium stearate, sodium stearyl fumarate, sodium stearyl lactylate, phosphatidylcholines, phosphatidylglycerols, talc, titanium dioxide, aluminium dioxide...

These excipients are anti friction agents (glidant) and will give better flow of powder in the dry powder inhaler which will lead to better dose reproducibility from the inhaler. They will tend to decrease the cohesion between the particles or will tend to improve the flow of powder in the inhaler. They decrease the resistance to sliding of the particles. Their addition will lead to improved release of the powder from the inhaler device and therefore better dose uniformity.

As indicated above, it is an object of the present invention that when the additive material is added, it is in a small amount. For example, magnesium stearate is highly surface active and should therefore be added in particularly small amounts like 5% (w/w) and preferably 2%(w/w) and more preferably less than 1% (w/w).

Contrary to the classical hydrophilic excipients generally used in DPIs (carbohydrates), the hydrophobic nature of the magnesium stearate associated with the active compound permits to reduce the absorption of the ubiquitous vapour leading to a reduction of the aggregation and the adhesion of particles. This improves the flowing property of the particles into the inhalation device, in particular during filling process, ensures accurate dosing of active ingredients and increases the dispersing property of cohesive dry particles during emission. The additive particle may be non-spherical.

Excipients can also be an amino acid. Amino acids may provide, when present in low amounts in the powders as additive material, high respirable fraction of the active materials with little segregation of the powder and also with very little of the amino acid being transported into the lower lung. The excipient may comprise one or more of any of the following amino acids: leucine, isoleucine, lysine, valine, methionine, phenylalanine. The additive may be a salt or a derivative of an amino acid, for example aspartame or acesulfame K.

In the present case, as indicated above, there will be an optimum amount of excipient, which amount will depend on the chemical composition and other properties of the carrier particles. However, it is thought that for most additives the amount of excipients in the powder (w/w) should be not more than 15%, more advantageously not more than 10%, preferably not more than 5% and for most materials will be not more than 2% or less by weight based on the weight of the powder.

Advantageously, the excipient consists of physiologically acceptable material. As already indicated, it is preferable for only small amounts of additive material to reach the lower lung, and it is also highly preferable for the excipient to be a material which may be safely inhaled into the lower lung where it may be absorbed in the blood stream. That is especially important where the excipient is in the form of particles.

Excipients, anti-friction agents, amino acids, nanoparticles of active compounds or excipient can be added to prevent the formation of stable agglomerates of particles, especially active particles, in the powder.

Advantageously, substantially all of the carrier particles have a diameter which lies between 20 µm and 1000 µm, more preferably 50 µm and 500 µm. Preferably, the diameter of substantially all of the carrier particles is less than 250 µm and lies between 20 µm and 200 µm. Preferably at least 90% by weight of the carrier particles have a diameter between from 1 µm to 100 µm.

In another possible embodiment, said active compound(s) can be coated by (or embedded in) said excipients and/or particles of the active drug in a continuous film and/or nanoparticles of the active drugs which are homogeneously distributed (i.e. homogeneously distributed in the coating layer).

This improves the flowing property of the particles into the inhalation device, in particular during filling process, ensures accurate dosing of active ingredients and increases the dispersing property of cohesive dry particles during emission.

However, it may be desirable for unstable (redispersible) agglomerates to be formed in the powder, and the size of the agglomerates may be as large as 10 µm or more. The size of particles in the powder, when considering the agglomerates, is to be taken as the size of the individual particles making up the agglomerate. The sizes of the individual particles and of the agglomerates may be determined using a suitable laser light diffraction techniques. For example, the higher compressed air values applied in the dispersion unit of the Mastersizer 2000^{®} (Malvern, U.K.) (up to 4 bar), permit all the agglomerates to break down, especially for micron-sized powders, as is the case for DPI formulations. In contrast, the air flow generated in the Spraytec^{®} (Malvern, U.K.), which simulates normal breathing conditions, is much lower and does not allow the de-agglomeration of particles. In other words, the Mastersizer 2000^{®} permit the determination of the size characteristics of totally individualized particles, whereas size results obtained from the Spraytec^{®} include the presence of agglomerates, probably corresponding to the population's finest particles.

The size of the particles may be determined using laser light diffraction, or other method in which the aerodynamic diameter of the particles may be determined, for example microscopic image analysis.

A laser light diffraction technique used a Malvern Spraytec^{®} (Malvern, U.K.) diffraction-based device equipped with an inhalation cell, specifically modified for measuring the particle size diameter (PSD) generated from medicinal aerosols, including MDI, DPI and nebulizers. It consists of a Spraytec^{®} unit with a throat held in place by the inhalation cell and a connection for a Multi-Stage Liquid Impinger (MsLi). The entire assembly is a closed system and allows for a controlled airflow rate (100 l/min during 2.4 sec.) in the measurement zone. This allows the size properties of DPIs to be measured under simulated breathing conditions.

Unless it is clear from the context (for example: mean particle size) otherwise, where reference is made to a range of sizes of particles, and to the size of particles, it is to mean that the majority of the relevant particles are within that range or that size (about at least 90 % by weight of the relevant particles will be in that range or in that size).

A method for preparing a composition according to the invention comprises the steps of:
(a) preparing a composition comprising:
   (i) particles in suspension in a liquid carrier, said particles containing at least one active compound; and
   (ii) a coating material comprising active material in suspension or solution in said liquid carrier; and
(b) spray drying said composition from step (a) to produce a dry powder composition wherein said particles of active compound are at least partially coated with said coating material.

In the case of suspensions, some components of the formulation can be partially or totally dissolved.

In a method according to the invention, for preparing said solution/suspension, an appropriate solvent system is chosen on the basis of the solubility of the different compounds._Water or any aqueous solution, ethanol, isopropanol and methylene chloride are examples of suitable solvent systems that can be used in a method of the invention. Any mixture of two, three, or more of said solvent systems can be used in a method of the invention.

The solvent system used can be heated in order to allow the dissolution of ingredient showing limited solubility characteristics. When the active substance(s), are not soluble in the solvent system chosen, a method for making a composition of the invention may further comprise, after the step of preparing a suspension containing the excipients and said active compound(s), and before the conversion step (spray-drying), a step of homogenizing said suspension.

A preferred process for converting said solution or suspension into particles consists of the spray drying process.

In a composition of the invention, it may be interesting to spray dry a suspension more than a solution in order to conserve the crystalline form of the particles. Spray-drying process does not affect the crystalline form of the active drug, which is very interesting in terms of guaranteeing the long term stability of the product.

Before spray-drying, a step of homogenization of the particles can be performed. Homogenization (micronization) is the process of reducing the particle sizes of pharmaceutical products, under very high pressures, sheer, turbulence, acceleration and impact, to make them more stable and clinically effective. The composition of the invention can be homogenized with an ultra-turrax at 24000 rpm during 10 minutes (in an ice bath to prevent sample temperature increase). The rotor acts as a centrifugal pump to recirculate the liquid and suspended solids through the generator, where shear, impact, collision and cavitation provide rapid homogenization. The volume of the suspension can be comprised between about 50 and 300 ml.

A composition of the invention can also be homogenized with a high pressure homogenizer. Homogenization is a fluid mechanical process that involves the subdivision of particles or droplets into micron sizes to create a stable dispersion or emulsion for further processing. It prepares feeds so that subsequent spray drying produces the best quality of powders. The fluid passes through a minute gap in the homogenizing valve. This creates conditions of high turbulence and shear, combined with compression, acceleration, pressure drop, and impact, causing the disintegration of particles and dispersion throughout the product. After homogenization, the particles are of a uniform size, typically from 0.2 to 2 micron, depending on the operating pressure. The homogenizer is the most efficient device for particle and droplet size reduction. The actual properties of the product vary with pressure and product type in a complex relationship. In general, higher processing pressure produces smaller particles, down to a certain limit of micronization.

The volume of suspension can be comprise between about 50 and 200 ml and the concentration of the active drug can be comprised between about 2 and 10% (w/v).

Pre-milling low pressure homogenization cycles are first conducted on the drug suspension to further decrease particle size (15 Cycles 7000 PSI - 10 Cycles 12.000 PSI). High pressure homogenization is then finally applied for 20 cycles at 24.000 PSI. Since HPH causes sample temperature increase, all operations are carried out using a heat exchanger, placed ahead of the homogenizing valve, with sample temperature maintained at 10 ± 1°C.

A surfactant can be added for nanoparticles stabilization after the homogenization operation, phospholipids, sodium glycocholate, SPAN 85 can be added to the suspension between 2 and 10% (w/w) as they are well-tolerated in the pulmonary tract.

Spray-drying is a one step process that converts a liquid feed (solution, coarse suspension, colloidal dispersion, etc.) to a dried particulate form.

The principal advantages of spray-drying with respect to a composition of the invention are the ability to manipulate and control particle size, size distribution, shape, and density in addition to macroscopic powder properties such as bulk density, flowability, and dispersibility.

With this method of production, it is also possible to coat or to embed the particles by pulverisation the suspension of the active drugs with a small amount of excipient or a fraction of the active drug in an amorphous state.

In classical spray dryers, the inlet and outlet temperatures are not independently controlled. Typically, the inlet temperature is established at a fixed value and the outlet temperature is determined by such factors as the gas flow rate and temperature, chamber dimensions, and feed flow rate.

A method of the invention can comprise a further step of heating the solution or suspension prepared, before the step of spray drying.

A method for preparing a composition according to the invention comprises the steps of:
- preparing a composition comprising particles in suspension in a liquid carrier, said particles containing at least one active compound and a coating material comprising active material in suspension or solution in said liquid carrier,
- optionally, reducing the size of the particles with high pressure homogenization,
- optionally, heating said solution or suspension to reach a temperature up to between 40°C and 70°C,
- in case of a suspension, homogenizing said suspension,
- converting the said solution or suspension into particles by feeding a spray drying system,
- spray drying the said solution or suspension.

A factorial design study can be used to determine the optimal conditions of spray-drying for the preparation containing said active compound(s) or said excipient(s) such as the spraying air flow, the drying air flow, the suspension feed rate, the nozzle size, the inlet and outlet temperature. When one or more ingredients of the composition are not soluble in the solvent system (i.e. suspension system), milling processes or high speed or high pressure homogenization techniques can be used in order to obtain appropriate particle size of the active compound or excipients prior to the step of conversion of a suspension to dried particles.

The invention also provides a composition comprising a liquid carrier, particles of active material in suspension in said liquid carrier and a coating material comprising active material in suspension and/or solution in said liquid carrier.

In a composition of the invention, the solid particles of excipients can be blended with the active drug. In this case, three different laboratory scale mixers namely Turbula 2C as a tumbling mixer, Collette MP-20 as a planetary mixer and Mi-Pro as a high shear mixer have shown quite satisfactory powder homogenisation results for mixing time comprised between about 10 minutes and about 60 minutes at optimal speeds. The dimensions of the combined active compound and excipient may still be within the optimum values for good deposition in the lower lung.

Advantageously, the particles obtained according to the invention present loose agglomerates with important dispersal properties during inhalation.

As indicated above, the exact ratio in which the active compound and the excipient are mixed will, of course, depend on the type of device and the type of active particles used. Also as indicated above, the proportion of the active compound in the powder is of particular importance. Advantageously, the powder comprises at least 80%, more preferably at least 90% by weight of active compound based on the total weight of the powder. Most advantageously, the powder comprises at least 95%, more preferably at least 98%, even more preferably 100%, by weight of active compound based on the weight of the powder.

Therefore, the quantities in which the excipient is added are preferably as small as possible.

A composition of the invention, in a powder form, may be used in a dry powder inhaler.

The new formulation according to the invention can be used in dry powder inhalers. Said dry powder inhaler can be for example a multidose system (reservoir system) or a monodose system, in which the powder is pre-packaged in either capsules (hard gelatine, hydroxypropylmethylcellulose (HPMC), or other pharmaceutically acceptable capsules) or in blisters.

The invention is described in further details in the following examples, which are intended for illustration purposes only, and should not be construed as limiting the scope of the invention in any way.

### Examples

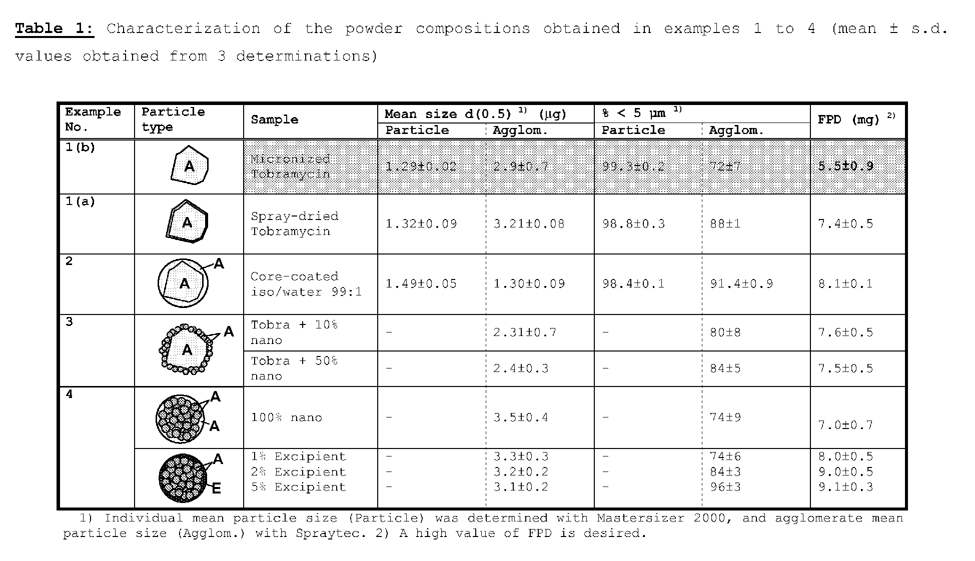

### Example 1: (a) Spray-dried (invention) vs (b) Micronized powders (comparative)

Example 1(a) illustrates the influence of the spray drying process on tobramycin particles for DPI inhalation.

The formulations were prepared, at laboratory scale, by spray-drying using a Büchi Mini Spray Dryer B-191a (Büchi laboratory-Techniques, Switzerland). The Mini Spray Dryer operates on the principle of nozzle-spraying in a parallel flow (the sprayed product and the drying air flow move in the same direction). Spray-drying has been recognized as a successful process to generate powders from solutions or suspensions in a single step. It converts a liquid feed (solution, coarse suspension, colloidal dispersion) to a dried particulate form. The principal advantages with respect to pulmonary drug delivery are the ability to manipulate and control particle size and size distribution, particle shape and density in addition to macroscopic powder properties such as bulk density, flowability, and dispersibility.

This Mini Spray-Dryer allows the recovering of a range of particle size going from 0.5 to 30 µm. The lower limitation is given by the separation of the cyclone used: smaller particles can not be separated anymore and are going into the filter.

In order to make a suspension, the solvent system chosen was isopropanol, which is a poor solvent for tobramycin. Tobramycin (5% w/v) was added in isopropanol and the suspension was homogenized with a CAT high speed homogenizer X620 (CAT M.Zipperer, Staufen, Germany) at 24000 rpm for 10 minutes. The suspension was then spray dried with constant stirring. The following conditions were used during spray drying: drying air flow, 35 m³/h; spraying air flow, 800 l/h; suspension feed rate, 2.7 g/min; nozzle size, 0.5 mm. The inlet temperature was established at 70° C and, in these conditions, the outlet temperature was about 35°C. The resultant powder was blown through the cyclone separator and collected in a container. Powders were stored in a dessicator at ambient temperature. The yield of the process which is the ratio of all the powder collected in the container and the total drug dose in suspension was about 80 %.

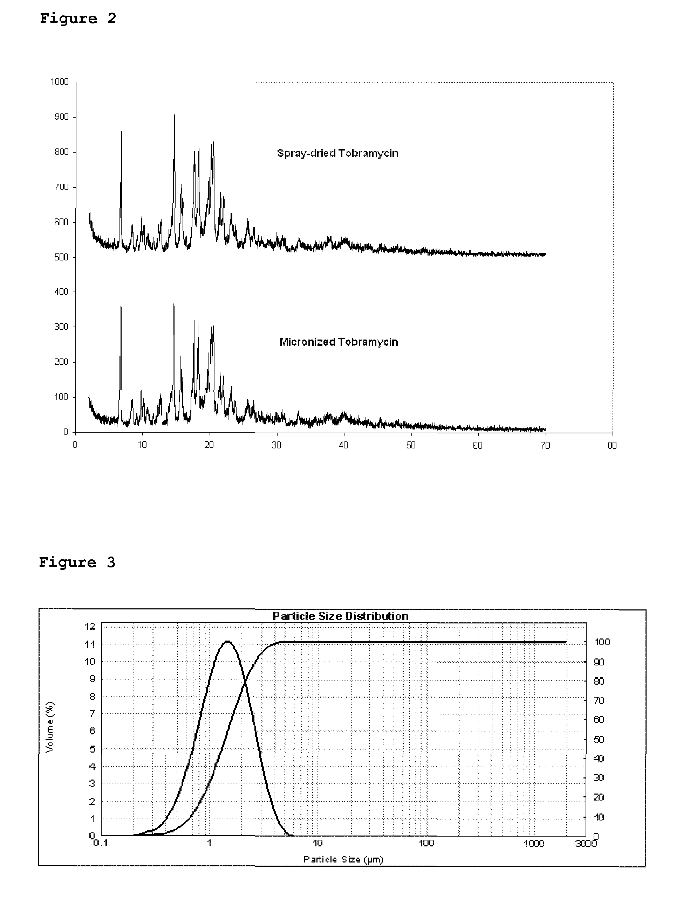

Figure 2 shows that the spray-drying process did not affect the crystalline form of tobramycin, which is very interesting in terms of guaranteeing the long term stability of the product. The peaks representing the lipid-coated spray-dried samples correspond to those for the original micronized tobramycin.

For each formulation, the particle size distribution is measured by laser diffractometry, using a dry sampling system with a suitable SOP (Standard Operating Procedure), (Scirocco®, Mastersizer 2000, Malvern, UK) and a Spraytec, (Malvern).

The size distributions are expressed in terms of the mass median diameter d(0.5) (= particle size corresponding to 50% cumulative distribution).

The particle size distribution measured with the Mastersizer 2000, Scirocco (Malvern) gives an indication of the size distribution of the individual particles of the powder composition as the powder agglomerates are broken down during the measuring process. On the other hand, the particle size distribution measured with the apparatus Spraytec (Malvern) gives an indication of the size of the powder agglomerates as they would appear at the exit of the dry powder inhaler (DPI). The latter measure is of prime importance as it strongly influences the amount of powder which will reach deep in the lungs.

Both Particle size distribution of formulation determined by using a Mastersizer 2000 and a Spraytec are illustrated in Table 1 (supra). The mass median diameter (d(0.5)) is low and is about 1.3 µm for both micronized and the spray dried tobramycin.

Figure 3 shows that spray-dried formulation presents a Gaussian curve (log normal distribution). The particle size distribution result obtained for the formulation is unimodal, narrow and ranges from 0.3 µm to 6 µm, with more than 90% of the particles having a diameter below 2.8 µm, which is required for an optimal deep lung deposition.

There were no major differences between spray-dried tobramycin of Example 1(a) and the micronized tobramycin of Example 1(b). So, spray-drying did not affect the particle size of the raw material.

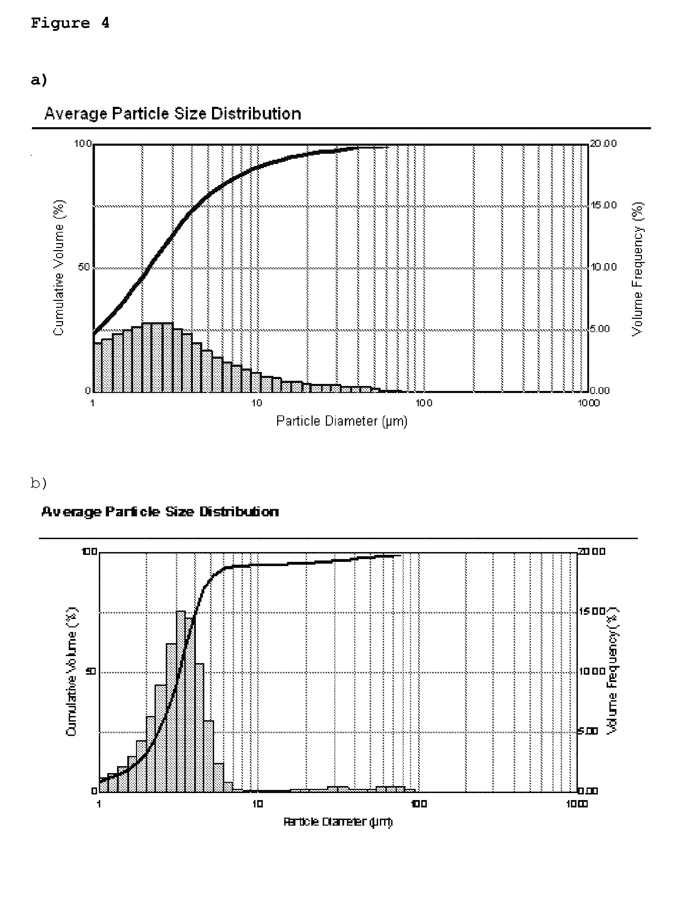

As shown in Table 1 (supra) and Figure 4, the desagglomeration tendency is higher for the spray-dried tobramycin with 88 % of particles having a diameter below 5.0 µm in comparison with the micronized tobramycin which presents 72 % of particles below 5.0 µm.

There is probably a problem of the raw powder agglomeration which is very prejudicial for pulmonary administration, decreasing the fine particle dose. As can be seen from these results, the spray drying of the active compound allows an improvement in the particle dispersion from the inhalator, thus enhancing the drug deposition deep in the lungs (loose agglomerates were easily scattered into small particles). There are more particles having a diameter in the respirable range i.e. between 1.0 and 5.0 µm in the spray-dried formulation. The principal advantage of spray-drying is the better desagglomeration tendency of the powder. Without wishing to be bound by any theory, the inventors explain the enhanced dispersion of spay-dried vs micronized powders by the partial dissolution of the surface layers of the active particles in isopropanol resulting, upon spray-drying of the powder, in the formation of a thin coat of active material onto the particles surface.

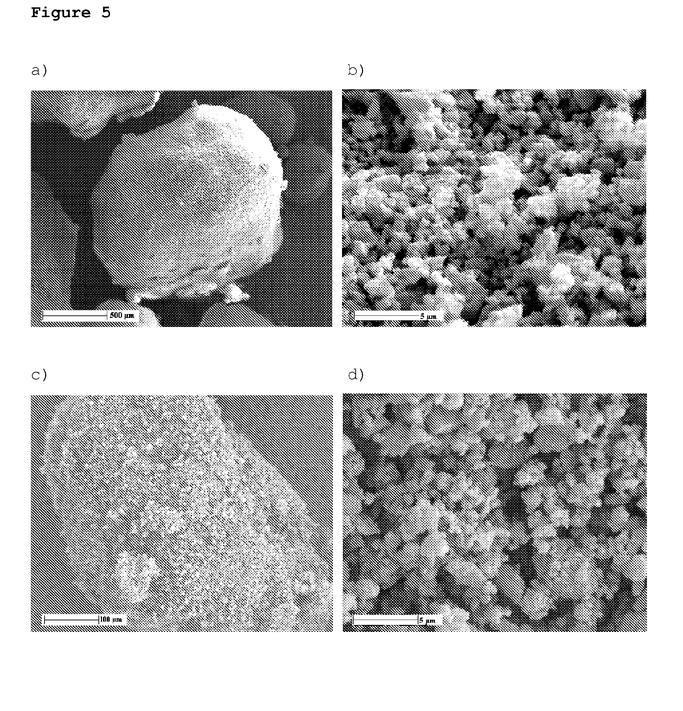

The morphology and surface structure of the formulations were analysed by SEM as shown in Figure 5. The bulk tobramycin of example 1(b) was formed by big, compact agglomerates of micron-sized particles. The size of agglomerates ranged up to 1 mm (cf. Fig. 5(a), (b)). The small tobramycin particles tended to form a very dense and cohesive structure. On the other hand, processing the suspensions by spray-drying (example 1(a)) yielded more regular-shaped and micron-sized particles. The powder formulation consisted of loose agglomerates (cf. Fig. 5(c)). At bigger magnifications, we can observe (cf. Fig. 5(d)) that agglomerates are composed of small smooth particles with a size of about 1-2 µm. The smoother particle surfaces probably explain the better dispersibility results obtained for the spray-dried formulation in comparison with the bulk tobramycin. Due to this morphology the particles became light, as was confirmed by the bulk density measurements, and the spray-dried formulations presented good flowability.

The Fine Particle Dose (FPD) has been determined by the method described in the European Pharmacopoeia 4 for the aerodynamic assessment of fine particle, using Apparatus C - Multi-stage Liquid Impinger (MsLI).

A dry powder inhalation device (Aerolyzer®, Novartis, Switzerland) was filled with a No. 3 HPMC capsule (Capsugel, France) loaded with 15 mg powder. The flow rate was adjusted to a pressure drop of 4 kPa, as typical for inspiration by a patient, resulting in a flow rate of 100 l/min during 2.4 seconds.

At least 3 Fine Particle Dose (FPD) determinations were performed on each formulation and analysis was carried out by a suitable and validated analytical HPLC method. In order to increase the UV absorptivity of the molecule, a derivatization method was applied. The suitable and validated quantification method is described in the USP 25.

The HPLC system consisted of a High Performance Liquid Chromatography system (HP 1100 series, Agilent technologies, Belgium), equipped with a quartenary pump, an autosampler and a variable wavelength UV detector set at 365 nm. The separation system was a 30 cm x 3.9 mm stainless steel (5 µm particle size) reversed-phase C18 column (Alltima, Alltech, Belgium). Samples of 20 µl volume were injected. The mobile phase was prepared by dissolving 2 g of Tris(hydroxymethyl) aminomethane in 800 ml of water. After this, 20 ml of H₂SO₄ 1 N was added and then the solution was diluted with acetonitrile to obtain 2 l, mixed and passed through a filter of 0.2 µm porosity. The flow rate was 1.2 ml/min.

The mass of test substance retained at each stage was determined from the HPLC analysis of the recovered solutions. Starting at the filter, a cumulative mass deposition (undersize in percentage) vs. cut-off diameter of the respective stages was derived and the FPD was calculated by interpolating the mass of active ingredient less than 5 µm.

The FPD is the dose (expressed in weight for a given nominal dose) of particles having an aerodynamic diameter inferior to 5 µm. It is considered to be directly proportional to the amount of drug able to reach the pulmonary tract in vivo, and consequently, the higher the value FPD, the higher the estimated lung deposition.

The Fine Particle Fraction (FPF) is the dose (expressed in weight %) of particles having an aerodynamic diameter inferior to 5 µm in relation to the nominal dose (FPD/loaded dose x 100).

The fine particle assessment results for the formulations are summarized in Table 2.

**Table 2: In vitro deposition study, with formulation given in example 1 vs. micronized tobramycin (raw material) (loaded dose=15mg, n=3).**

| | **Micronized** | **Spray-dried** |
|---|---|---|
| | **tobramycin** | **tobramycin** |
| **Device** | 6.0 ± 0.7 | 4.8 ± 0.4 |
| **Throat** | 0.3 ± 0.1 | 0.36 ± 0.06 |
| **Stage 1** | 0.9 ± 0.3 | 0.7 ± 0.1 |
| **Stage 2** | 0.6 ± 0.4 | 0.8 ± 0.1 |
| **Stage 3** | 2.1 ± 0.9 | 2.9 ± 0.3 |
| **Stage 4** | 1.7 ± 0.6 | 2.9 ± 0.1 |
| **Filter** | 1.4 ± 0.3 | 1.7 ± 0.1 |
| **FPD** | 5.5 ± 0.9 | 7.4 ± 0.1 |
| **Metered dose** | 12.9 ± 0.3 | 14.2 ± 0.7 |

The FPD, which is around 5.5 mg for the micronized tobramycin, is increased by up to about 7.4 mg for the spray-dried formulation of the invention, in terms of deep lung penetration.

The results of the present example show that the spray dried coated powder compositions yield better results than the micronized ones. This new coated tobramycin powder DPI formulation with 100% drug level presents high lung deposition properties and offers perspectives in improving the delivery of high doses of drugs to the pulmonary tract. These formulations are more particularly useful for drugs that are active at relatively high doses, such as antibiotics, as they permit the delivery of a high concentration of antibiotic directly to the site of infection while minimizing systemic exposition. A reduction in administration time and in systemic side effects allows improved suitability of these formulations for patients.

The flow properties of active powder compositions can be enhanced by coating with an excipient the particles of the powder compositions as taught in US-A-2005/0152849, but the amount of active compound contained in one inhaled powder dose is thus reduced by the presence of relatively large amounts of excipient. By incorporating active compound in the coating allows to yield in combination good flow properties with higher doses of active compound than possible in US-A-2005/0152849.

### Example 2 (Invention)

In order to dissolve a small fraction of the tobramycin particles, water has been added to the suspension of isopropanol. In this isopropanol/water mix, it was demonstrated that the majority of tobramycin is present as suspended products and a small amount of tobramycin is dissolved. Without being linked to any particular theory, it is hypothesized that the dissolved particles coat the active particles of tobramycin and modify the properties of the surface, the flowability and the aerolization of the active drug particles.

The method carried out for preparing the active compound comprises the steps of:
- preparing a solution of isopropanol and water in an appropriate ratio (isopropanol/water 99:1) .
- Adding the micronized tobramycin (5% w/v) and homogenizing the suspension during 10 minutes with a CAT high speed homogenizer X620 at 24000 rpm and
- spray drying the resulting suspension with constant stirring, using the Büchi mini spray dryer B-191 (Büchi laboratory-Techniques, Switzerland) to form particles. The following conditions were used during spray drying: drying air flow, 35 m³/h; spraying air flow, 800 l/h; suspension feed rate, 2.7 g/min; nozzle size, 0.5 mm. The inlet temperature was established at 200° C and, in these conditions, the outlet temperature was about 100°C. The resultant powder was blown through the cyclone separator and collected in a container. Powders were stored in a dessicator at ambient temperature.

The particle size distribution and the Fine Particle Dose are determined as mentioned in comparative example
1. The results are shown in Table 1 (supra).

As it can be observed from Table 1 (supra), the mass median diameter of the individual particles as measured with the Mastersizer device is about 1.5 µm, i.e., larger than the uncoated spray dried particles of comparative example 1, which would suggest lower FPD values for the coated powder particles. The results of Table 1, however, clearly show that the coated particles have a higher FPD value than the uncoated particles of comparative example 1. This result can be explained by the lower size of the powder agglomerates as evidenced by the lower mean size measured with the Spraytec device (1.3 vs. 3.2 µm for coated and uncoated powders, respectively).

The particle size distribution results obtained for the different formulations are unimodal with 91% of the particles having a diameter below 5.0 µm, which corresponds to upper size limits required for an optimal deep lung deposition.

The fine particle assessment results for the formulations, represented by the FPD, are summarized in Table 1 (supra).

The FPD is about 8.1 mg for the formulation containing 1 % of water. The presence of 1 % of water involving the coating of the active particles with a small fraction of the dissolved particles, increases the fine particle dose, permitting a better desagglomeration of the powder.

### Example 3

In this example, nanoparticles of the active drug are used to increase the desagglomeration of the tobramycin powder.

After dispersion of tobramycin in isopropanol, a first size-reduction step using a CAT high speed homogenizer X620 at 24000 rpm (10 min for a 50 ml sample) is conducted on the suspension (in an ice bath to prevent sample temperature increase). Nanosuspensions are then prepared using an EmulsiFlex-C5 high pressure homogenizer (Avestin Inc., Ottawa, Canada). Pre-milling low pressure homogenization cycles are first conducted on the tobramycin suspension to further decrease particle size (15 cycles 7000 PSI - 10 cycles 12.000 PSI). High pressure homogenization is then finally applied for 10 to 20 cycles at 24.000 PSI. Since HPH causes sample temperature increase (increase of 30°C following 20 cycles at 24.000 PSI), all operations are carried out using an heat exchanger, placed ahead of the homogenizing valve, with sample temperature maintained at 10 ± 1°C. Samples are withdrawn after the different size reduction steps for size distribution analysis (cf. Table 3).

**Table 3: Particle size distribution of suspension of tobramycin 5% (w/v) determined by using a Mastersizer 2000 with wet sampling systems (Hydro 2000), (mean ± s.d. values obtained from 3 determinations, n=3)**

| | **d(0.1)** | **d(0.5)** | **d(0.9)** |
|---|---|---|---|
| **Suspension Tobra** | 0.54 ± 0.03 | 1.57 ± 0.05 | 20.67 ± 0.05 |
| **After Turrax** | 0.22 ± 0.02 | 0.96 ± 0.09 | 4.62 ± 0.05 |
| **After hph 12000 PSI** | 0.11 ± 0.01 | 0.50 ± 0.02 | 3.60 ± 0.02 |
| **After hph 24000 PSI** | 0.09 ± 0.01 | 0.40 ± 0.01 | 1.22 ± 0.01 |

Through the results obtained, we can clearly see that further processing of the pre-milled drug suspensions allows for greater particle size reduction with achievement of a population with a d(0.5) around 0.40 µm. The particle size decreases when increasing the pressure of the cycles.

A surfactant can be necessary to improve nanoparticle stabilization after the homogenization operation. So, phospholipids, sodium taurocholate or sodium glycocholate can be added at 1, 2, 5 and 10 % w/w to the suspension before homogenization in order to yield the results in regards to particle size reduction efficiency.

**Table 4: Particle size distribution of suspension of tobramycin 5% (w/v) and surfactant 2% (w/w) after 10 cycles of 12000 PSI and 10 cycles of 24000 PSI, determined by using a Mastersizer 2000 with wet sampling systems (Hydro 2000)(mean ± s.d. values obtained from 3 determinations, n=3)**

| | **d(0.1)** | **d(0.5)** | **d(0.9)** |
|---|---|---|---|
| **With Phospholipon** | 0.08 ± 0.01 | 0.31 ± 0.01 | 1.195 ± 0.02 |
| **With Na taurocholate** | 0.09 ± 0.01 | 0.29 ± 0.01 | 1.39 ± 0.02 |
| **With Na glychocolate** | 0.08 ± 0.01 | 0.21 ± 0.01 | 1.22 ± 0.01 |

A sodium glychocolate concentration as low as 2% (w/w relative to tobramycin) was shown sufficient for suspension preparation and stabilization as it can be seen from the results presented in table 4. This showed a particle size distribution characterized by d(0.5) around 0.21 µm.

The method carried out for preparing the final suspensions for spray-drying containing nanoparticles comprises the steps of:
- Adding micronized tobramycin (2, 5 and 10 % w/v) to isopropanol and then adding the nanoparticles (5, 10 and 50 % w/w relative to tobramycin). The suspension was then homogenized with a CAT high speed homogenizer X620 at 24000 rpm for 10 minutes.
- spray drying the resulting suspension with constant stirring, using the Büchi mini spray dryer B-191 (Büchi laboratory-Techniques, Switzerland) to form particles. The following conditions were used during spray drying: drying air flow, 35 m³/h; spraying air flow, 800 l/h; suspension feed rate, 2.7 g/min; nozzle size, 0.5 mm. The inlet temperature was established at 70° C and, in these conditions, the outlet temperature was about 35°C. The resultant powder was blown through the cyclone separator and collected in a container. Powders were stored in a dessicator at ambient temperature.

The particle size distribution results obtained for the different formulations are unimodal and, as shown in Table 1 (supra), more than 80% of the particles have a diameter below 5.0 µm, which corresponds to upper size limits required for an optimal deep lung deposition.

To characterize the nanoparticle fraction after spray-drying, tobramycin powder was poured into isopropanol and centrifuged at 3000 rpm during 5 min in order to isolate the nanoparticle fraction.

**Table 5: Particle size distribution of the nanoparticle fraction of the spray-dried suspension of tobramycin 5% (w/v) and tobramycin nanoparticles 50% (w/w relative to tobramycin), determined by using a Mastersizer 2000 with wet sampling systems (Hydro 2000) (mean ± s.d. values obtained from 3 determinations, n=3)**

| | **D(0.1)** | **D(0.5)** | **D(0.9)** |
|---|---|---|---|
| **Nanoparticles of Tobra with 50% nano** | 0.12 ± 0.01 | 0.32 ± 0.01 | 0.78 ± 0.01 |

As shown in Table 5 and Figure 6, the particle size analysis shows that the spray-drying process has no significant effect on the average size of the nanoparticles.

The fine particle assessment results for the formulations, represented by the FPD, are summarized in Table 1 (supra).

The FPD, which is around 7.4 mg for the Spray-dried tobramycin, is increased by up to about 7.6 mg for the nano-coating formulation, in terms of deep lung penetration. The presence of the nanoparticles decreased the agglomeration tendency of a spray dried powder. Coating of the fine drug particles with particles in the nanometer range might reduce Van Der Waals forces and powder agglomeration. The principal advantage is that the micronized particles are coated with active compounds and not with excipients which might be less tolerated in the pulmonary tract. So, addition of nanoparticles can improve the flowability and increase the FPD of the powder.

### Example 4

In this example, dry powders were produced by spray-drying suspension containing 100% of nanoparticles of tobramycin. After dispersion of tobramycin 5% w/v, a first size-reduction step using a CAT high speed homogenizer X620 at 24000 rpm (10 min for a 50 ml sample) is conducted on the suspension (in an ice bath to prevent sample temperature increase). Nanosuspensions are then prepared using an EmulsiFlex-C5 high pressure homogenizer (Avestin Inc., Ottawa, Canada). Pre-milling low pressure homogenization cycles are first conducted on the tobramycin suspension to further decrease particle size (10 cycles 12.000 PSI). High pressure homogenization is then finally applied for 10 cycles at 24.000 PSI. All operations are carried out using a heat exchanger, placed ahead of the homogenizing valve, with sample temperature maintained at 10 ± 1°C.

Immediately following the reduction size step, the resulting suspension was spray dried with constant stirring, using the Büchi mini spray dryer B-191 (Büchi laboratory-Techniques, Switzerland) to form particles. The following conditions were used during spray drying: drying air flow, 35 m³/h; spraying air flow, 800 l/h; suspension feed rate, 2.7 g/min; nozzle size, 0.5 mm. The inlet temperature was established at 70° C and, in these conditions, the outlet temperature was about 35°C. The resultant powder was blown through the cyclone separator and collected in a container. Powders were stored in a dessicator at ambient temperature.

Sodium glycocholate was added at 1, 2 and 5 % w/w relative to tobramycin to the suspension before homogenization in order to yield the results in regards to particle size reduction efficiency and to stabilize the suspension.

As shown in Table 1, the desagglomeration tendency seems higher for the nanoparticles with 2 and 5% of sodium glycocholate with 84 and 96 % of particles having a diameter below 5.0 µm in comparison with the micronized tobramycin which presents 72 % of particles below 5.0 µm.

As can be shown from these results, the spray-drying of suspension of nanoparticles allowed an improvement in the particle dispersion from the inhalator, thus enhancing the drug deposition deep in the lungs (loose agglomerates were easily scattered into small particles).

The FPD, which is around 5.5 mg for the micronized tobramycin, is increased by up to about 9 mg for the nanoparticles formulation, in terms of deep lung penetration. It can be seen that the presence of sodium glycocholate allows an increase in the fine particle deposition. It tends to reduce the nanoparticles aggregation and consequently gives a better deep lung deposition.

### Example 5

This example illustrates the influence of the spray drying process on nacystelyn particles for DPI inhalation. The formulations were prepared, at laboratory scale, by spray-drying using a Büchi Mini Spray Dryer B-191a (Büchi laboratory-Techniques, Switzerland).

A suspension of nacystelyn (2, 5 or 10 % w/v) was added in isopropanol which is a poor solvent for nacysterlyn and the suspension was homogenized with a CAT high speed homogenizer X620 at 24000 rpm for 10 minutes. The suspension was then spray dried with constant stirring. The following conditions were used during spray drying: drying air flow, 35 m³/h; spraying air flow, 800 l/h; suspension feed rate, 2.7 g/min; nozzle size, 0.5 mm. The inlet temperature was established at 70° C and, in these conditions, the outlet temperature was about 35°C. The resultant powder was blown through the cyclone separator and collected in a container. Powders were stored in a dessicator at ambient temperature.

This manipulation allows the control of particle size and size distribution, particle shape and density in addition to macroscopic powder properties such as bulk density, flowability, and dispersibility. The spray-drying of the active particles allows an improvement in the particle dispersion from the inhalator, thus enhancing the drug deposition deep in the lungs.

### Example 6

In order to dissolve a higher fraction of the nacystelyn particles than in Example 5, water has been added to the suspension of isopropanol. The dissolved and amorphous particles coat the active particles of nacystelyn and modify the properties of the surface, the flowability and the aerolization of the active drug particles. The different solvent systems were composed of isopropanol:water 80:20, 90:10, 95:5, 99:1.

The method carried out for preparing the active compound comprises the steps of:
- preparing a solution of isopropanol and water in an appropriate ratio.
- Adding the nacystelyn (2, 5 or 10 % w/v) and homogenizing the suspension during 10 minutes with a CAT high speed homogenizer X620 at 24000 rpm and
- spray drying the resulting suspension with constant stirring, using the Büchi mini spray dryer B-191 (Büchi laboratory-Techniques, Switzerland) to form particles. The following conditions were used during spray drying: drying air flow, 35 m³/h; spraying air flow, 800 l/h; suspension feed rate, 2.7 g/min; nozzle size, 0.5 mm. The inlet temperature was established at 200° C and, in these conditions, the outlet temperature was about 100°C. The resultant powder was blown through the cyclone separator and collected in a container. Powders were stored in a dessicator at ambient temperature.

The presence of the amorphous particles in a continuous film decreased the agglomeration tendancy of the raw powder. Coating of the fine drug particles with a continuous film might reduce Van Der Waals forces and powder agglomeration. The principal advantage is that the micronized particles are coated with active compounds and not with excipients which might be less tolerated in the pulmonary tract.

### Example 7

This example illustrates the influence of the spray drying process on budesonide particles for DPI inhalation. The formulations were prepared, at laboratory scale, by spray-drying using a Büchi Mini Spray Dryer B-191a (Büchi laboratory-Techniques, Switzerland).

In order to make a suspension, the solvent system chosen was water which is a poor solvent for budesonide.

A suspension of budesonide (2, 5 or 10 % w/v) was added in water and the suspension was homogenized with a CAT high speed homogenizer X620 at 24000 rpm for 10 minutes. The suspension was then spray dried with constant stirring. The following conditions were used during spray drying: drying air flow, 35 m³/h; spraying air flow, 800 l/h; suspension feed rate, 2.7 g/min; nozzle size, 0.5 mm. The inlet temperature was established at 150° C and, in these conditions, the outlet temperature was about 75°C. The resultant powder was blown through the cyclone separator and collected in a container. Powders were stored in a dessicator at ambient temperature.

This manipulation allows the control of particle size and size distribution, particle shape and density in addition to macroscopic powder properties such as bulk density, flowability, and dispersibility. The spray-drying of the active particles allows an improvement in the particle dispersion from the inhalator, thus enhancing the drug deposition deep in the lungs.

### Example 8

In order to dissolve a higher fraction of the budesonide particles than in Example 7, isopropanol has been added to the suspension of water.

The dissolved particles coat the active particles of budesonide and modify the properties of the surface, the flowability and the aerolization of the active drug particles.
The different solvent systems were composed of water:isopropanol 50:50, 70:30, 80:20, 90:10.

The method carried out for preparing the active compound comprises the steps of:
- preparing a solution of isopropanol and water in an appropriate ratio.
- Adding the budesonide (2, 5 or 10 % w/v) and homogenizing the suspension during 10 minutes with a CAT high speed homogenizer X620 at 24000 rpm and
- spray drying the resulting suspension with constant stirring, using the Büchi mini spray dryer B-191 (Büchi laboratory-Techniques, Switzerland) to form particles. The following conditions were used during spray drying: drying air flow, 35 m³/h; spraying air flow, 800 l/h; suspension feed rate, 2.7 g/min; nozzle size, 0.5 mm. The inlet temperature was established at 150° C and, in these conditions, the outlet temperature was about 75°C. The resultant powder was blown through the cyclone separator and collected in a container. Powders were stored in a dessicator at ambient temperature.

The presence of the amorphous particles decreased the agglomeration tendancy of the raw powder. Coating of the fine drug particles with a continuous film might reduce Van Der Waals forces and powder agglomeration. The principal advantage is that the micronized particles are coated with active compounds and not with excipients which might be less tolerated in the pulmonary tract.

### Example 9

In order to confirm the promising in vitro deposition test results, one composition was selected on the basis of the aerodynamic behaviour and compared to Tobi^{®} (nebulizer solution) by an in vivo scintigraphic evaluation and a pharmacokinetic study of the bioavailability of inhaled tobramycin after a single oral dose in nine cystic fibrosis patients.

The formulation consisted of micronized tobramycin coated by particles of the active drug in an amorphous state which are homogeneously distributed in the coating layer. The powder was obtained by spray-drying a mixture of isopropanol:water 98:2 containing micronized tobramycin 5% w/v (see example 2). Size #3 HPMC capsules (Capsugel, Bornem, Belgium) were filled with 25 mg of this powder.

The second formulation was the comparator product, the Tobi^{®} (nebulizer solution). The study design was an open single-dose, cross-over study with a 7 days wash-out period between the each phase of the study. Approvals were obtained from the Ethics Comitee of Erasme Hospital (Ref.: P2006/072) and the Belgian Minister of Social Affairs and Public Health (Ref.: EudraCT n° 2006-000456-40).

Scintigraphic images of the chest and lateral oropharynx were recorded immediately after the drug inhalation (DHD-SMV, Sopha Medical, France). The empty device, capsule, mouthpiece and exhalation filter were also counted.

Venous blood samples were collected at pre-dose and at 30 min, 1 h, 1.5 h, 2 h, 2.5 h, 3 h, 4 h, 6 h, 8 h and 10 h post-dose in order to quantify plasma levels of tobramycin. The concentration of tobramycin was measured using a validated LC/MS-MS method (High Performance Liquid Chromatography (HP 1100 series, Agilent Technologies, Belgium) and API 365 quadrupole mass spectrometer (Applied Biosystems/MDS Sciex, Concord, Canada).

For the formulation, the radiolabel (^{99m}Tc) was adsorbed on the active particles in isopropanol. This is achieved by wetting the drug particles with a nonsolvent containing the radiolabel, followed by the evaporation of the solvent (spray-drying), leaving the radiolabel on the surface of the drug particles.

Nebulized tobramycin was radiolabelled by simply dissolving an appropriate amount of ^{99m}Tc-DTPA (diethylene triamine penta-acetic acid) complex in the aqueous nebulised tobramycin solution.

In order to demonstrate the quality of the radiolabelling method, i.e. the particle physical characteristics were not modified by the radiolabelling and the radiolabel was effectively deposited at he surface of particles; experiments were carried out prior to the clinical part of the investigation. For each formulation, the particle size of the unlabeled drug (n=3) was determined and compared against the particle size distribution of the radiolabelled drug (n=3) and of the radiolabel (n=3). The measurements were made with a Multistage Liquid Impinger (MsLI, Copley instruments, U.K.) operating at an airflow rate corresponding to a pressure drop of 4kPa over each inhaler (Eur. Ph. 5^{th} edition). The test was carried out at 100 L/min during 2.4 secondes. Drug and radiolabel content were determined by a validated analytical HPLC method and by gamma counting (Cobra gamma counter, Packard Bioscience, UK), respectively. Quantification of tobramycin was done by the US Pharmacopeia method that involves derivatization of the tobramycin with 2,4-dinitrofluorobenzene, and quantification by high-performance liquid chromatography. The fraction of drug or radiolabel, corresponding (by interpolation) to particles having an aerodynamic diameter inferior to 5µm, was defined as the Fine Particle Dose (FPD) and was calculated as percentage of the nominal dose.

For nebulized tobramycin, the radiolabel and drug are both dissolved in the aqueous phase, and hence the concentration of drug and radiolabel were uniformly distributed in a given nebulized droplet.

The distribution of the drug (before and after labelling) and of the radiolabel are summarized in figure 7. The results did not show any significant differences among the three measurements on any stages of the MsLI. The amount remaining in the device was also similar for the three measurements. The FPF values (mean ± SD) obtained for the drug before labelling (57.6 ± 2.3%), after labelling (56.5 ± 2.8%) and for the radiolabel (56.1 ± 2.9%) were not significantly different (*p* > 0.05).

These validation experiments demonstrated that the PSD of the radiolabel and the drug alone were well matched, with no alteration of aerosol properties of the formulations after radiolabelling. So, for both formulations a homogenous cover of ^{99m}Tc around the active drug that did not affect the size of the particles was observed.

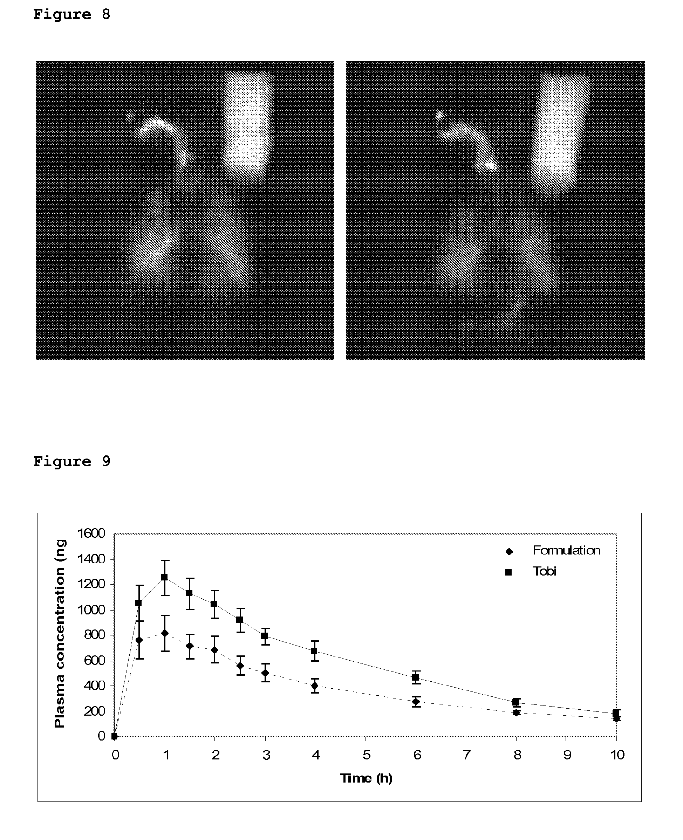

Scintigraphic images showing deposition patterns of the upper part of the body of one subject for the formulation and the comparator product are shown in Figure 8.

The fractionation of the delivered dose between the whole lungs, oropharynx, inhaler device and exhalation filter for the two products is shown table 6.

**Table 6: Mean fractionation (± SD (CV)) of the dose between lungs, oropharynx, stomach and device for the DPI tobramycin and the Tobi^{®}.**

| | **Tobra DPI** | **Tobi** |
|---|---|---|
| **Lungs** | 34.1 ± 12.4 (36) | 7.6 ± 2.7 (39) |
| **Oropharynx** | 15.2 ± 7.1 (47) | 2.7 ± 1.3 (50) |
| **Stomach** | 2.1 ± 2.0 (93) | 1.8 ± 1.2 (68) |
| **Device** | 43.4 ± 14.6 (33) | / |

Mean ± SD lung deposition for Tobra DPI was 34.1 ± 12.4% (ranged from 15.3 to 50.0%), for an overall CV of 36%.These results corresponded to 8.5 mg tobramycin deposited in the lung, assuming a total delivered dose of 25 mg via the Aerolizer^{®}. The lung deposition in patients was lower than the in vitro FPF, indicating, as expected, that the nature of the disease and, probably, its severity play key roles in the bioavailability of drugs in the lungs.

Concerning the reference product (Tobi^{®}), after a time of 20 min, necessary to nebulize to dryness the product, only 7.6 ± 2.7% (CV of 39%) of the radiolabelled tobramycin (range from 3.6% to 13.2%) was deposited in the lung and 2.7 ± 1.3% in the oropharynx. This result corresponded to 22.8 mg tobramycin deposited in the lung, assuming a total delivered dose of 300 mg after nebulization.

Lung deposition was thus estimated to be 4.5 times higher for the tobramycin DPI than for the Tobi^{®}. The mean lung deposition for tobramycin DPI was significantly greater (p < 0.05) than the mean lung deposition of the Tobi^{®}.

The quantification of plasma levels of tobramycin (normalized plasma concentration-time profile based on the actual lung dose of the formulations) is illustrated in Figure 9.

As can be seen in Table 7, the mean AUC and Cₘₐₓ of the Tobi^{®} were approximately 4 times higher than those of Tobra DPI. Nevertheless, the dose administrated of Tobi^{®} was 12 times greater than for the DPI form (300 mg vs. 25 mg). Only one inhalation was performed and the DPIs were loaded with about twelve fold less drug, but there is no reason why multiple inhalations could not be performed.

**Table 7: Mean ± SD (CV) pharmacokinetic parameters for tobramycin (n=9)**

| | **Tobra DPI** | **Tobi** |
|---|---|---|
| **Tmax (h)** | 1.2 ± 0.5 (43) | 0.9 ± 0.3 (32) |
| *Parameters* | | |
| **AUC (ng x h/ml)** | 1348 ± 622 (46) | 5687 ± 1590 (28) |
| **C max (ng/ml)** | 331 ± 184 (56) | 1274 ± 389 (30) |
| *Adjusted for the same lung dose* | | |
| **AUC (ngx h/ml)** | 3615 ± 1669 (46) | 5687 ± 1590 (28) |
| **C max (ng/ml)** | 887 ± 494 (56) | 1274 ± 389 (30) |

However, when adjusted to the same drug dose of the comparator product (Tobi^{®}) deposited in the lung (based on scintigraphic deposition values (22.8 mg)), the same proportion of the drug that was delivered to the lung did not reach the systemic circulation for the nebulization and the DPIs. This can be explained by differences in the physical state (solution vs. solid form) of the two forms, leading to a much slower rate of dissolution in bronchial fluid (delay in the Tₘₐₓ). The particles of tobramycin from the DPI forms seem to present a greater retention in pulmonary tissue than the tobramycin droplets, which is beneficial for the treatment of the CF patient, decreasing the systemic exposure.

The examination of figure 7, representing the plasma concentration-time profile adjusted to the same drug dose of the comparator product (Tobi^{®}) deposited in the lung (based on scintigraphic deposition values (22.8mg)), showed a rapid plasma concentration peak for the DPI formulation as well as for the comparator product, followed by a progressive decrease in tobramycin plasma concentrations over 10 hours. Nevertheless, Tobi^{®} (nebulizer solution) was absorbed a little faster than the Tobra DPI with maximum plasma concentrations (Cₘₐₓ) occurring at 0.9 h and 1.2 h, respectively. So, the droplets with their hydrophilic properties and a median diameter about 2.2 µm were dissolved more easily than the particles of tobramycin in the bronchial fluid and then in the systemic circulation.

As can be observed from the examination of the pharmacokinetic data adjusted to the same drug dose of the comparator product (Tobi^{®}) deposited in the lung, shown in Table 7, the Cₘₐₓ and AUC values were found to be significantly higher for Tobi^{®} than for DPI formulations (*p <* 0.05). For example, the Cₘₐₓ values were found to be 1274 ng/ml and 887 ng/ml for the Tobi^{®} and Tobra DPI, respectively.

Consistent with the difference in Cₘₐₓ between the two methods of administration, the AUC after inhalation of the nebulised tobramycin was 1.6 times greater that after Tobra DPI (5687,3 ng x h/ml vs. 3615 ng x h/ml, p < 0.01).

Thus, the DPI formulation, based on the pharmacokinetic results, showed the advantages of greater local retention with reduced systemic absorption and side effects. Like other aminoglycosids, tobramycin has a narrow safety margin and systemic exposure may cause severe ototoxicity and nephrotoxicity.

## Claims

1. A spray dried powder for use in dry inhalers, comprising particles (**A**) comprising active material, said particles being at least partially coated (**C₁, C₂**) with an active material so that the flowing and dispersing properties of the powder are enhanced.

2. A powder according to claim 1, wherein said active material comprises at least one medical drug suitable for being administered nasally or orally.

3. A powder according to claim 1 or 2, wherein the composition comprises a combination of two or more active compounds.

4. A powder according to any of the preceding claims, wherein the coating material (**C₁, C₂**) comprises the same active compound as the particles (**A**).

5. A powder according to any of claims 1 to 3, wherein the coating material (**C₁, C₂**) comprises active compounds different from the ones contained in said particles (**A**).

6. A powder according to any of the preceding claims, wherein the coating forms a continuous film (**C₁**).

7. A powder according to any of claims 1 to 5, wherein the coating is discrete and comprises nanopaticles (**C₂**) of at least one active compound.

8. A powder according to any of the preceding claims, wherein the mean size of the coated particles is at most about 20 µm, preferably at most 10 µm, 5 µm, and more preferably at most 1 µm, or 0.2 µm.

9. A powder according to any of the preceding claims, wherein the active compounds are selected from the group consisting of antibiotics, anti-histaminic, anti-allergic agents, antimicrobial agents, antiviral agents, anticancer agents, antidepressants, antiepileptics, antipains, cromones, mucolytic, leukotrienes, leukotriene antagonist receptors, muscle relaxants, hypotensives, sedatives, therapeutic proteins, peptides and genes, (poly)peptides, gamma, growth hormones, colony stimulating factors, TNFs, blood factors, enzymes, antibodies, virus infections, alpha-1-antitrypsin, proteoglycans, or a mixture of thereof.

10. A powder according to claim 9, wherein said antibiotics comprise macrolides such as azithromycin and clarithromycin or aminoglycosides such as tobramycin.

11. A powder according to claim 9 or 10, wherein said mucolytic comprises nacystelyn.

12. A powder according to any of the preceding claims, wherein the coating (**C₁, C₂**) further comprises excipients.

13. A powder according to any of the preceding claims, wherein the composition contains at least 80% by weight of active compound compared to the total weight of the powder composition.

14. A powder according to claim 12 or 13, wherein the coating (**C₁, C₂**) comprises nanoparticles (**C₂**) of excipients.

15. A powder according to claim 13 or 14, wherein the coating comprises no active ingredient.

16. A powder according to any of claims 12 to 15, wherein said excipient comprises a glidant material, a carrier material, amino acids, derivative of an amino acid, or a combination thereof.

17. A powder according to claim 16, wherein the composition comprises not more than about 5%, preferably not more than 2% and more preferably not more than 1% by weight of said glidant material.

18. A powder according to claim 16 or 17, wherein said glidant material comprises magnesium stearate.

19. A powder according to claim 16 or 19, wherein the composition comprises not more than 10% by weight, preferably not more than 5% by weight, of carrier material.

20. A powder according to claim 16, wherein the carrier material is selected from monosaccharides, disaccharides, polyols derived from these disaccharides, such as lactitol, mannitol, or their monohydrates ; polysaccharides, inorganic salts such as sodium chloride and calcium carbonate; organic salts such as sodium lactate; other organic salts such as urea, polyssacharides; oligosaccarides such as cyclodextrins and dextrins; sorbitan esters; aerosil 200; lecithin; lauric acid, palmitic acid, stearic acid, erucic acid, behenic acid, or derivatives; sodium stearyl fumarate, sodium stearyl lactylate; phosphatidylcholines, phosphatidylglycerols and other examples of natural and synthetic lung surfactants, lauric acid and its salts, for example, sodium lauryl sulphate, magnesium lauryl sulphate, triglycerides, sugar esters; phospholipids, cholesterol; talc; titanium dioxide, aluminium dioxide, silicone dioxide and starch or a mixture of thereof.

21. A powder according to claim 16, wherein said amino acid comprises one or more of any of the following amino acids: leucine, isoleucine, lysine, valine, methionine, phenylalanine.

22. A powder according to any of the preceding claims, wherein the particle (**A**) of active material are microparticles.

23. A powder according to any of the preceding claims, wherein the particle (**A**) of active material are nanoparticles.

24. A method for preparing a powder, comprising the following steps:
(a) preparing a composition comprising:
(i) particles (**A**) in suspension in a liquid carrier, said particles containing at least one active compound; and
(ii) a coating material (**C₁, C₂**) comprising active material in suspension and/or solution in said liquid carrier; and
(b) spray drying said composition from step (a) to produce a dry powder composition wherein said particles (**A**) of active compound are at least partially coated with said coating material (**C₁, C₂**).

25. A method according to claim 24, wherein the size of the particles (**A**) is controlled by a milling process or high speed or high pressure homogenization techniques prior to the spray-drying step.

26. A method according to claim 24 or 25, wherein said active particles (**A**) have a mean size of at most 20 µm, preferably at most 5 µm, more preferably at most 3 µm or at least 2 µm.

27. A method according to any of claims 24 to 26, wherein at least 95% by weight of active particles (**A**) have a size lower 5 µm.

28. A method according to any of claims 24 to 27, wherein the method includes the steps of heating said solution or suspension to reach a temperature comprised between 40°C and 70°C.

29. A method according to any of claims 24 to 28, wherein the method includes the steps of homogenizing said suspension before converting it into a dry powder by said spray drying step (b).

30. A method according to claim 29, wherein after homogenization, the particles (**A**) are of a uniform size between 0.2 and 2 µm.

31. A method according to claim 24, wherein the particle (**A**) of active material are nanoparticles.

32. A method according to any of claims 24 to 31, wherein said liquid carrier is selected from the group consisting of water or any aqueous solution, ethanol, isopropanol, methylene chloride and hexane.

33. A method according to claim 32, wherein said liquid carrier comprises a mixture of different solvents or co-solvents.

34. A method according to any of claims 24 to 33, wherein the coating material (**C₁, C₂**) comprises the same active compound as said particles (**A**).

35. A method according to any of claims 24 to 33, wherein the coating material (**C₁, C₂**) comprises an active compound different from the one contained in the particles (**A**).

36. A method according to any of claims 24 to 35 wherein at least one excipient is dissolved in said liquid carrier.

37. A method according to any of claims 24 to 36, wherein the coating material comprises nanoparticles (**C₂**).

38. The method according to any of claims 24 to 37 wherein the liquid carrier contains excipient nanoparticles (**C₂**) in suspension therein.

39. A method according to claim 27, 37 or 38, wherein the nanoparticles have a mean size comprised between 0.001 and 1 µm, preferably between 0.002 µm and 0.5 µm.

40. A method according to any of claims 27, 37 to 39, wherein said nanoparticles are produced by high pressure homogenization.

41. The method according to any of claims 37 to 40, wherein the coating material (**C₁, C₂**) contains no active compound.

42. A method according to claim 37 to 38, wherein the particles (**A**) essentially have a mean size in the range of 0.5 to 10 µm, preferably in the range of 1 µm to 5 µm.

43. A composition comprising a liquid carrier, particles (**A**) containing active material in suspension in said liquid carrier and a coating material (**C₁, C₂**) comprising active material in suspension and/or solution in said liquid carrier.

44. A dry powder inhaler containing a powder according to any of claims 1 to 23.

45. A dry powder inhaler according to claim 44, wherein said powder comprises a mixture of at least two active materials.

46. A dry powder inhaler according to claim 44 or 45, wherein at least 5 mg of at least one active material is dispensed by one actuation of the inhaler.

47. A dry powder inhaler according to any of claims 44 to 46, wherein at least 5 mg of each of the active materials contained in said powder is dispensed by one actuation of the inhaler.
